# EUROPEAN PATENT APPLICATION

(11) **EP 4 635 625 A1**
(43) Date of publication of application: **22.10.2025**
(21) Application number: 23902653.7
(22) Date of filing: 11.12.2023
(51) Int. Cl.: B01J 38/02, B01J 23/843, B01J 23/94, B09B 3/40, C07C 29/42, C07C 33/046, B09B 101/95

(54) **REGENERATION METHOD FOR CATALYST FOR PREPARING 1,4-BUTANEDIOL BY MEANS OF ALKYNE HYDROFORMYLATION REACTION**

(30) Priority: 12.12.2022 CN 202211599664
(71) Applicant: BASF Corporation, Florham Park, NJ 07932 (US)
(72) Inventor: WANG, Huan, Shanghai 200137 (CN); LV, Ai Ling, Shanghai 200137 (CN); SOORHOLTZ, Mario, Shanghai 200137 (CN)
(74) Representative: Maiwald GmbH
(86) International application number: PCT/CN2023/137906
(87) International publication number: WO 2024/125457

(57) **Abstract**

A regeneration method for a catalyst, specifically relating to a regeneration method for a copper-bismuth catalyst for preparing 1,4-butynediol by means of reaction of formaldehyde and acetylene in a slurry reactor.

## Description

### Technical Field

The present invention relates to a regeneration method for a catalyst, and specifically to a regeneration method for a copper-bismuth catalyst for preparing 1,4-butynediol by means of reaction of formaldehyde and acetylene in a slurry reactor.

### Background Art

In the 1940s, Reppe invented a process of synthesizing 1,4-butynediol using formaldehyde and acetylene as raw materials. In this process, a heavy metal of copper acetylide is used as a catalyst, and the reaction is carried out in a fixed-bed reactor with a higher pressure, which increases the operational risk of acetylene and copper acetylide. After the 1970s, a new type of copper-bismuth catalyst was developed. This catalyst is small particle and has good activity, which can reduce the operation pressure in a reaction in a slurry reactor, reducing the risk of explosion and thereby improving the Reppe process. Currently, the slurry reactor process has become one of the main methods for synthesizing 1,4-butynediol due to its advantages, such as fast synthesis reaction speed, low pressure, safe operation, and convenient catalyst replacement.

However, in the actual production process, the activity of the aforementioned copper-bismuth catalyst will gradually decrease with prolonged use time due to the influence of impurities in raw materials, operation conditions, and polyalkyne by-products. Thus, on the one hand, the yield of the product will be affected, and on the other hand, the aggregation of polyalkyne by-products results in increased viscosity of the system, thereby reducing the service life and filtering effect of filtering equipment. The overall activity of the catalyst system is maintained mainly by discharging a part of the spent catalyst and supplementing a fresh catalyst. The main ingredients of the spent catalyst are copper and bismuth metals, residual carriers, residual formaldehyde and 1,4-butynediol, and a large amount of polyalkyne substances covering the surface of the catalyst. In current treatment methods, the concentrated spent copper-bismuth catalyst is generally compacted, then stacked as solid waste, and sold to waste metal recycling enterprises for copper recovery. However, due to the residual copper acetylide substance in the spent catalyst, it is extremely sensitive to heat and shock after drying, resulting in a low safety factor. In addition, this recycling method is time-consuming, consumes a large amount of strong acid, has high recycling costs, and is prone to causing severe environmental pollution. Therefore, there is an urgent need to solve the problem of how to safely and controllably recover and regenerate copper-bismuth catalysts at low costs.

CN 1048919 C discloses a regeneration method for a copper-bismuth catalyst for synthesizing 1,4-butynediol via a reaction in a slurry reactor, in which a spent catalyst is oxidized with a strong oxidant of sodium hypochlorite in a liquid phase, followed by separation and drying to obtain a regenerated catalyst, which can achieve a catalytic performance similar to an original fresh catalyst. However, this method may easily cause the loss of copper in the solid phase system, and the introduced chlorine may easily deactivate the regenerated catalyst. During the same period, CN 1048920 C discloses another regeneration method for the spent copper-bismuth catalyst, in which a spent catalyst is reacted with a formaldehyde solution, followed by separation, washing, drying, and then roasting in the air or oxygen to obtain a regenerated catalyst. However, this treatment method will introduce additional formaldehyde-containing wastewater and requires a long time to completely treat the copper acetylide complex. CN 105413711 A discloses a regeneration method for a spent copper-bismuth catalyst under a mild condition, in which water-seal suction filtration is used, and with a copper ion protectant added, a filter cake formed by the spent catalyst is subjected to two-step liquid phase oxidation with an aqueous hydrogen peroxide solution, followed by separation and drying to obtain a regenerated catalyst. However, this patent does not disclose relevant activity data, so it is still unknown whether the regenerated catalyst can meet the actual production requirements. CN 108067240 B discloses a secondary treatment of a spent catalyst by using various composite solvents to dissolve most of the organic matter covering the surface of the catalyst, followed by roasting at a low temperature to restore the activity of the deactivated catalyst. It avoids the severe aggregation of metal oxides caused by high-temperature roasting that affects the activity and stability of the regenerated catalyst. Specifically, the spent catalyst is dissolved with nitric acid and then subjected to a coprecipitation reaction again, during which a special preparation process is used so that the prepared catalyst has higher activity and stability. Before roasting, the spent catalyst is subjected to secondary treatment with a composite surfactant, thus reducing the consumption of nitric acid and also the content of impurities in the catalyst and improving the stability of the catalyst. During the same period, CN 108069825 B discloses that in the invention, the organic matter covering the surface of the catalyst is effectively removed online by using a surfactant and an alkali solution, and then sulfur and phosphorus in cuprous sulfide and cuprous phosphide deposited on the surface of the catalyst are removed in the form of hydrogen sulfide and hydrogen phosphide in an air atmosphere or a mixed atmosphere of hydrogen and air, thereby restoring the activity of the catalyst. Due to the use of a strong oxidant, or the involvement of a complicated washing operation, etc., none of the patents above can be easily applied to the actual recovery of large quantities of spent catalysts in industry.

### Summary of the Invention

The present invention proposes a regeneration method for a spent copper-bismuth catalyst from the synthesis of 1,4-butynediol by means of reaction of formaldehyde and acetylene in a slurry reactor.

In an aspect, the present invention relates to a regeneration method for a spent copper-bismuth catalyst, including:
step 1) of subjecting a spent catalyst containing copper and bismuth to solid-liquid separation to obtain a solid mass;
step 2) of drying the solid mass obtained in step 1) to obtain a dry product; and
step 3) of calcining the dry product of step 2) to obtain a regenerated catalyst.

In another aspect, the present invention relates to a regenerated catalyst obtained according to the method described above.

In still another aspect, the present invention relates to a method for using the regenerated catalyst for hydrogenation, dehydrogenation, hydrogenolysis, or ethynylation.

### Detailed Description of the Invention

Before explaining several exemplary embodiments of the present invention, it should be understood that the present invention is not limited to the details of the construction or process steps mentioned in the description below. The present invention may have other embodiments and can be implemented or carried out in various ways.

For the terms used in this disclosure, the following definitions are provided.

Throughout the specification, including the claims, unless otherwise stated, the terms "comprising/including a" or "comprising/including an" should be understood as synonymous with the term "comprising/including at least one", and "between...and..." or "...to..." should be understood to be inclusive of the defined values.

The terms "a", "an" and "the" are used to refer to one or to more than one (i.e., to at least one) of the grammatical objects of the article.

The term "and/or" includes the meanings "and", "or", and also all other possible combinations of elements associated with the term.

Unless otherwise stated, all the percentages and ratios are mentioned by weight.

Therefore, according to an aspect of the present invention, a method for regenerating a spent copper-bismuth catalyst is provided, which includes:
step 1) of subjecting a spent catalyst containing copper and bismuth to solid-liquid separation to obtain a solid mass;
step 2) of drying the solid mass obtained in step 1) to obtain a dry product; and
step 3) of calcining the dry product of step 2) to obtain a regenerated catalyst.

In one or more embodiments, the fresh catalyst for a reaction in a slurry reactor is a supported mixture of copper oxide and bismuth oxide. During the reaction in the slurry reactor, the copper oxide reacts with formaldehyde and acetylene to generate an active substance of copper acetylide, which plays a catalytic role in the synthesis of 1,4-butynediol. As the reaction proceeds, polyalkyne substances will be continuously generated on the surface of the catalyst and gradually accumulate over time, which will reduce catalytic activity, resulting in the deactivation of the catalyst.

In one or more embodiments, the inorganic substances in the spent copper-bismuth catalyst comprise 5-65 wt%, preferably 10-55 wt%, more preferably 15-50 wt%, including 25, 30, 35, 40 and 45 wt% of Cu, calculated as CuO; 0.1-10 wt% of Bi, preferably 0.2-7 wt%, more preferably 0.3-5 wt%, including 0.4, 1.0, 1.5, 2.0, 2.5, 3.0, 3.5, 4.0 and 4.5 wt% of Bi, calculated as Bi₂O₃; and 40-95 wt%, preferably 50-90 wt%, including 55, 60, 65, 70, 75, 80 and 85 wt% of a carrier.

In one or more embodiments, the carrier may include, but is not limited to, silicon oxide, magnesium oxide, magnesium silicate, alumina, calcium oxide, activated carbon, and a mixture thereof.

The term "mixture" or "combination" used herein relates to but is not limited to, any combination of physical or chemical forms, such as a blend, a solution, a suspension, an alloy, a composite, and the like.

In one or more embodiments, the carrier is alumina, the weight of which is calculated as Al₂O₃.

The alumina typically used herein may be *α* -alumina, *β* -alumina, *γ* -alumina, and a mixture thereof.

In one or more embodiments, the drying is carried out at a temperature of 10-150°C, including 20°C, 30°C, 40°C, 50°C, 60°C, 70°C, 80°C, 90°C, 100°C, 110°C, 120°C, 130°C and 140°C.

In one or more embodiments, the calcination is carried out at a temperature of 300-700°C, including 400°C, 500°C and 600°C.

In one or more embodiments, in step 1), the spent catalyst containing copper and bismuth does not react with an aqueous formaldehyde-containing solution and is directly subjected to solid-liquid separation.

In one or more embodiments, in step 2), the solid mass obtained in step 1) is directly dried.

In the method described above, the deactivated catalyst can be treated using a simple and mild method to remove the polyalkyne substances on the surface thereof; meanwhile, copper oxide and bismuth oxide are generated during the reaction, thus regenerating the catalyst.

Another aspect of the present invention provides a regenerated catalyst obtained according to the method described above.

Other aspects include methods for using the regenerated catalyst described above for hydrogenation, dehydrogenation, hydrogenolysis, or ethynylation.

The advantages of the present invention are low energy consumption and less pollution during treatment, convenience for large-scale industrial promotion, and that it is economical.

Various embodiments are described below. It should be noted that the specific embodiments are not intended to be an exhaustive description or a limitation on broader aspects discussed herein.

### Detailed Description of Embodiments

### Activation and test of catalytic performance of catalysts

The performance of the regenerated catalyst is evaluated using an activation-reaction two-step method. A catalyst to be tested is mixed with an aqueous formaldehyde solution in a first reactor. The catalyst is activated after introducing an acetylene stream and heating the reactor from room temperature to about 80°C, with the pH controlled in a range of 7.0-10.0, preferably 8.5, during the activation process. The activation process usually requires 5 hours. Then, the slurry is removed, centrifuged and decanted, leaving the wet catalyst for the activity test. A certain amount of the wet catalyst is mixed with the aqueous formaldehyde solution in a second reactor. Then, a stream of acetylene is introduced at a partial pressure of typically 0.5-1.9 atm, preferably 1.0 atm. The catalyst is present in an amount of about 1-20 parts by weight per 100 parts by weight of an aqueous formaldehyde medium. The reactor is heated from room temperature to about 80°C. The reaction process usually requires 5 hours. The product mixture is analyzed via gas chromatography to quantify the main product of 1,4-butynediol therein, and thus the generation rate of butynediol is calculated to evaluate the activity of the catalyst.

The present invention will be further explained below in conjunction with specific examples.

### Example 1

A fresh supported copper-bismuth catalyst 1 (containing 34.9% of copper oxide, 0.7% of bismuth oxide, and 64.4% of alumina) was prepared by a method according to the patent CN 111939919 A. The supported copper-bismuth catalyst was used to catalyze a reaction of formaldehyde and acetylene at 80°C for 10 h, and the spent catalyst was then removed and concentrated by means of centrifugal separation. The supernatant was removed to obtain the lower concentrate, and the concentrate was left to stand at 50°C for drying and then calcined at 500°C for 1 h. On conclusion of the calcination, a regenerated supported copper-bismuth catalyst was obtained, which was referred to as a regenerated catalyst 1A.

### Comparative Example 1

A fresh supported copper-bismuth catalyst 1 (containing 34.9% of copper oxide, 0.7% of bismuth oxide, and 64.4% of alumina) was prepared by a method according to the patent CN 111939919 A. The supported copper-bismuth catalyst was used to catalyze a reaction of formaldehyde and acetylene at 80°C for 10 h, and the spent catalyst was then removed and treated by the method according to CN1048920C. Specifically, the spent catalyst was added to a 160 ml container with a stirrer, and 80 ml of a 37% formaldehyde solution was added for reaction at 80°C for 10 h. Then, the resulting slurry was concentrated by means of centrifugal separation. The supernatant was removed, and the concentrate was washed with water. After centrifugal separation again, the washing liquid was removed to obtain the lower concentrate, and the concentrate was left to stand at 50°C for drying and then calcined at 500°C for 1 h. On conclusion of the calcination, a regenerated supported copper-bismuth catalyst was obtained, which was referred to as a regenerated catalyst 1B.

### Comparative Example 2

A fresh supported copper-bismuth catalyst 2 (containing 46.7% of copper oxide, 2.9% of bismuth oxide, 43.1% of silicon oxide, and 7.3% of magnesium oxide) was prepared by a method according to the patent US 9308522 B2. The supported copper-bismuth catalyst was used to catalyze a reaction of formaldehyde and acetylene at 80°C for 10 h, and the spent catalyst was then removed and concentrated by means of centrifugal separation. The supernatant was removed to obtain the lower concentrate, and the concentrate was left to stand at 50°C for drying and then calcined at 500°C for 1 h. On conclusion of the calcination, a regenerated supported copper-bismuth catalyst was obtained, which was referred to as a regenerated catalyst 2A.

The composition information of the regenerated catalysts and fresh catalysts is shown in table 1. The results show that for the alumina-supported copper-bismuth catalyst, the regenerated one has approximately the same composition.

**Table 1. Comparison of composition of fresh catalysts and regenerated catalysts[*]**

| | CuO, wt% | Bi₂O₃, wt% | Al₂O₃, wt% | SiO₂, wt% | MgO, wt% |
|---|---|---|---|---|---|
| Fresh catalyst 1 | 34.9% | 0.7% | 64.4% | / | / |
| Regenerated catalyst 1A | 32.1% | 0.6% | 67.3% | / | / |
| Regenerated catalyst 1B | 32.9% | 0.7% | 66.4% | / | / |
| Fresh catalyst 2 | 46.7% | 2.9% | / | 43.1% | 7.3% |

| | | | | | |
|---|---|---|---|---|---|
| [*] The composition data are normalized | | | | | |

### Catalyst activity evaluation

The catalytic activity of the regenerated catalysts obtained in the examples and comparative examples above were tested, respectively, and the detailed procedures were shown below. Activation was carried out in a reactor containing 100 mL of formalin (a 37 wt% aqueous formaldehyde solution). A 1.5 M sodium hydroxide solution was added to the formalin to adjust the initial pH to about 8.0, and at the end of the pH adjustment, 15 g of a catalyst was added to the formalin. The reactor was inertized via nitrogen purging, and then the gas stream was replaced with 80 mL/min acetylene. At a controlled pH of 8.0, stirring was started, followed by heating until the temperature reached 80°C. The reaction was maintained for 5 h. Then, the reactor was cooled to room temperature under the gas stream of acetylene. The reactor was flushed with nitrogen for inertization, and the slurry was removed, centrifuged, and decanted, leaving the wet catalyst for the activity test. 0.8 g (on a dry basis) of the catalyst was added to a reactor charged with an aqueous formaldehyde solution. Similarly, the initial pH of formalin was adjusted to 8.0 with a sodium hydroxide solution. The flow rate of acetylene was kept constant at 50 mL/min, and the reaction temperature was 80°C. After 5 hours, the reactor was cooled under the gas stream of acetylene and then flushed with nitrogen for inertization. The slurry was removed and centrifuged. The product mixture was analyzed via gas chromatography to quantify butynediol therein. Thus, the activity of the catalyst was evaluated by calculating the generation rate of butynediol, with 300 min reaction time and 0.8 g catalyst. The catalyst information described above is shown in Table 2.

**Table 2. Evaluation data of catalyst activity**

| | Generation rate of butynediol (mol/min/g_{cat}) | Ratio of generation rate of butynediol (regenerated catalyst/fresh catalyst) |
|---|---|---|
| Fresh catalyst 1 | 1.20E-04 | 100% |
| Regenerated catalyst 1A | 0.57E-04 | 47.5% |
| Regenerated catalyst 1B | 0.29E-04 | 24.2% |
| Fresh catalyst 2 | 1.21E-04 | 100% |
| Regenerated catalyst 2A | 0.07E-04 | 5.8% |

For the alumina-supported copper-bismuth catalyst, the activity of the catalyst regenerated using the method of the present invention is approximately 47.5% of that of a fresh catalyst. Through comparison, it can be found that the activity of this regenerated catalyst is about 23% higher than that of the regenerated catalyst obtained using the formaldehyde-copper acetylide reaction method described in CN 1048920 C. Furthermore, the regeneration process involved in the present invention is simple, with less pollution and low energy consumption, convenient for large-scale industrial promotion, and economical. For the magnesium silicate-supported copper-bismuth catalyst, the activity of the catalyst regenerated using the method of the present invention is 5% of that of a fresh catalyst.

## Claims

1. A method for regenerating a spent copper-bismuth catalyst, comprising:
step 1) of subjecting a spent catalyst containing copper and bismuth to solid-liquid separation to obtain a solid mass;
step 2) of drying the solid mass obtained in step 1) to obtain a dry product; and
step 3) of calcining the dry product of step 2) to obtain a regenerated catalyst.

2. The method according to claim 1, wherein the inorganic substances in the spent copper-bismuth catalyst comprise 5-65 wt% of Cu, calculated as CuO; 0.1-10 wt% of Bi, calculated as Bi2O3; and 40-95 wt% of a carrier.

3. The method according to claim 2, wherein the carrier is Al2O3.

4. The method according to any one of claims 1-3, wherein the drying is carried out at a temperature of 10-150°C.

5. The method according to any one of claims 1-4, wherein the calcination is carried out at a temperature of 300-700°C.

6. The method according to any one of claims 1-5, wherein in step 1), the spent catalyst containing copper and bismuth does not react with an aqueous formaldehyde-containing solution and is directly subjected to solid-liquid separation.

7. The method according to any one of claims 1-6, wherein in step 2), the solid mass obtained in step 1) is directly dried.

8. A regenerated catalyst obtained by the method according to any one of claims 1-7.

9. A method for using the regenerated catalyst of claim 8 for hydrogenation, dehydrogenation, hydrogenolysis, or ethynylation.
